Europäisches Patentamt

European Patent Office (11) Numéro de publication: **0 004 514**

Office européen des brevets **B1**

(19)

(12) **FASCICULE DE BREVET EUROPEEN**

(45) Date de publication du fascicule du brevet: **27.04.83**

(51) Int. Cl.³: **A 61 N 1/04**

(21) Numéro de dépôt: **79400186.7**

(22) Date de dépôt: **21.03.79**

(54) **Electrode cutanée pour l'application sur le corps humain de courants électriques de traitement thérapeutique et esthétique.**

(30) Priorité: **28.03.78 FR 7808845**

(43) Date de publication de la demande:
**03.10.79 Bulletin 79/20**

(45) Mention de la délivrance du brevet:
**27.04.83 Bulletin 83/17**

(84) Etats contractants désignés:
**BE CH DE FR GB IT LU NL SE**

(73) Titulaire: **Guillot, Jacqueline Galie**
**F-31510 Barbazan (FR)**

(72) Inventeur: **Guillot, Jacqueline Galie**
**F-31510 Barbazan (FR)**

(74) Mandataire: **Foldès, Georges et al,**
**CABINET J. BONNET THIRION 95 Bd.**
**Beaumarchais**
**F-75003 Paris (FR)**

(56) Documents cités:
**DE - A - 2 414 584**
**DE - A - 2 618 947**
**DE - C - 811 709**
**DE - C - 892 944**
**DE - C - 943 610**
**FR - A - 2 271 846**
**US - A - 3 315 665**
**US - A - 3 565 059**
**US - A - 4 016 869**
**US - A - 4 125 110**

Courier Press, Leamington Spa, England.

Electrode cutanée pour l'application sur le corps humain de courante électriques de traitement thérapeutique ou esthétique

L'invention se rapporte à un moulage électroconducteur destiné à servir d'électrode de contact en forme avec la peau du corps humain en sorte de lui appliquer, dans des buts de traitement thérapeutique ou esthétique, des courants électriques de forme et sens déterminé, délivrés par un générateur approprié.

Les méthodes de traitement pas passage de courant par la peau du corps humain sont connues en soi et en dehors du cadre de la présente invention. On fait passer les courants dans la peau entre une ou une pluralité d'électrodes cutanées, en général formées de plaques conductrices rigides, ou élastiquement déformables, reliées à une première borne d'un générateur prévu pour engendrer des courants de forme et sens définis par la méthode de traitement, et une ou une pluralité de contre-électrodes reliées à une seconde borne du générateur. Les contre-électrodes peuvent être analogues aux électrodes cutanées, ou constituées par des tampons souples imbibés d'un liquide conducteur physiologiquement neutre. L'action des courants pénétrant dans la peau sous les électrodes cutanées est souvent renforcée par onction de la peau avec des compositions pharmacologiquement actives, tandis que les contre-électrodes utilisées pour le retour des courants vers le générateur ont un comportement essentiellement neutre.

On précisera que les électrodes cutanées considérées ici se distinguent fondamentalement d'électrodes utilisées comme prises de contact destinées à l'enregistrement de signaux électriques d'origine organique, tels que des signaux de cardiogrammes ou d'encéphalogrammes, ces prises de contact étant très localisées et prévues pour transmettre des signaux de puissance minime, tandis que les électrodes cutanées considérées peuvent intéresser des régions étendues du corps et doivent transmettre à l'organisme des puissances non négligeables, avec des densités superficielles de puissance équilibrées dans toutes les régions intéressées. Alors que les prises de contact doivent viser essentiellement à minimiser les potentiels de contact, les électrodes cutanées considérées doivent viser la répartition équilibrée des courants dans les régions intéressées, dans des conditions où les potentiels de contact ont une influence négligeable.

Les électrodes cutanées courantes présentent l'inconvénient que les plaques, même élastiques, n'épousent pas rigoureusement tous les contours de la peau, surtout si les régions intéressées présentent des saillies ou des concavités accusées, ou des arêtes osseuses ou encore des parties délicates ou sensibles, où l'application de pressions capables d'amener en conformation réciproque la peau et l'électrode est inacceptable. Les traitements du visage sont de ce point de vue particulièrement difficiles. Or le traitement de la peau n'est efficace que là où elle est franchement au contact de l'électrode cutanée, de sorte qu'avec les électrodes courantes il y a un risque d'irrégularités de traitement avec des zones à frontières visibles, aggravé éventuellement par un danger de brûlures locales en des zones de concentration excessive de courant. Par ailleurs le traitement de larges étendues de peau, par exemple de la taille aux chevilles, exige une multiplicité d'électrodes, difficiles à placer et fixer contiguës, et dont le couplage convenablement équilibré au générateur nécessite un réseau de conducteurs encombrant et difficile à régler.

La demande de brevet français N° 75 15622 présente des compositions d'électrodes conçues pour être appliquées directement sur la peau de malades aux fins de stimulation électrique pour agir sur la douleur et la motricité, compositions qui comportent un liant résineux auquel est mélangé une certaine quantité de poudre conductrice de l'électricité, et se rapprochent des peintures conductrices utilisées pour des applications industrielles électriques. Les liants résineux sont choisis tels que, dans les conditions d'utilisation, notamment en solution, ils ne soient ni toxiques, ni irritants, ni allergéniques et adhèrent à la peau, dans trois classes comprenant des résines acrylovinyliques, des uréthannes polyéther et des acétates de polyvinyle, tandis que les poudres conductrices sont des paillettes d'argent, d'or ou de platine, ou une poudre de graphite. Les compositions sont mises en place normalement sous forme de peintures avec des solvants dermatologiquement acceptables tels que l'acétone, l'éthanol, l'isopropanol et analogues. En variante, les compositions acrylovinyliques chargées de poudre d'argent peuvent réaliser des enductions adhésives sur des feuilles de papier ou de métal.

En réunissant des enseignements épars dans la demande précitée, on comprend que les électrodes cutanées forment des pellicules très souples, d'épaisseur comprise entre quelques micromètres et quelques dizièmes de millimètre, qui sont prévues pour occuper des zones localisées du corps, telles que des articulations, typiquement le poignet, et y séjourner plusieurs jours (au minimum trois jours) de façon à pouvoir exécuter une série de séances de traitement. L'adhérence des pellicules conductrices est conditionnée par un dégraissage préalable de la peau. De plus les électrodes cutanées ainsi réalisées sont prévues pour des traitements relativement localisés, ce qui results du fait que la peau doit être tendue pour l'application, ce qui ne peut être exécuté que dans des zones restreintes. Par ailleurs, l'application de films continus, même possédant une certaine preméabilité à la vapeur d'eau, sur des régions étendues du corps et pendant plusieurs jours consécutifs serait de nature à provoquer des lésions cutanées.

On conçoit donc que les électrodes cutanées décrites dans la demande N° 75 15622 ne sont en général pas aptes à être substituées aux électrodes constituées de plaques rigides ou élastiques mentionnées précédemment dans l'exposé de l'état de la technique pour l'application de méthodes de traitement thérapeutique ou esthétique par passage de courants dans la peau auxquelles se réfère la présente invention, et qui visent des traitements de la peau ou des tissus sous-jacents, sur des régions étendues du corps, incluant le visage.

L'invention a pour objet une électrode cutanée, adaptée à l'application des méthodes précitées, capable d'épouser tous les contours de la peau sur de larges étendues du corps humain, en sorte de permettre une répartition équilibrée des courants de traitement dans toute l'étendue de la peau épousée.

L'invention a également pour objet une électrode cutanée du genre précédent, qui assure, durant son application, un maintien général de la forme de la peau avec une certaine souplesse permettant de petites déformations de la peau sans perte de contact.

L'invention a encore pour objet une électrode cutanée capable de servir de support à des adjuvants de traitement.

A ces effets l'invention propose une électrode cutanée pour application sur la peau du corps humain de courants électriques de traitement thérapeutique ou esthétique, en association avec une contre-électrode, électrode et contre-électrode étant couplées respectivement à deux bornes d'un générateur de courants électriques de traitement, électrode du genre que l'on prépare sous forme d'une composition à appliquer directement sur la peau, se solidifiant après mise en place et constituée par un mélange d'un liant plastique et d'un additif conducteur d'électricité, caractérisée en ce que le liant plastique est constitué d'une poudre susceptible de prise avec l'eau, et d'eau en quantité supérieure à celle qui se combine avec la poudre durant la prise, l'additif est un sel dissous dans l'eau ajoutée pour la prise, la composition en se solidifiant formant un moulage électroconducteur qui épouse et maintient la forme du corps.

On connaît la fidélité et la finesse de détail des moulages réalisés avec une poudre capable de prise avec l'eau, dont le type est le plâtre, couramment utilisé pour des moulages anatomiques. L'incorporation dans l'eau de prise d'un sel de calcium soluble donne au moulage une conductivité électrique due à l'ionisation du sel en présence de l'eau de prise en excès sur la quantité entrant en réaction avec la poudre au cours de la prise.

Pour établir des connexions de couplage au générateur de courant, il est préféré d'incorporer un contact métallique au moulage, en l'enrobant dans le mélange poudre-eau de prise pendant l'exécution du moulage.

On utilisera de préférence un plâtre de catégorie dentaire, c'est-à-dire un plâtre commercialisé pour les moulages dentaires, et une eau de prise en quantité voisine en masse de la moitié de celle du plâtre, et contenant en solution entre 3 et 10% en masse de chlorure de calcium. On obtient ainsi par gâchage une pâte permettant une application aisée, et dont la vitesse de prise et l'élévation de température concomitante sont ajustables, en fonction des conditions de traitement choisies, par ajustement de la quantité de chlorure de calcium mise en oeuvre.

En solution préférée, l'eau de prise contient en outre un produit tensio-actif protéiné et des adjuvants actifs sur la peau. Le produit tensio-actif a tendance à former une mousse avec l'eau de prise, favorisant ainsi une structure spongieuse du moulage, qui supporte mieux de faibles déformations. La structure spongieuse favorise également une migration des adjuvants et composants protéinés vers la peau du sujet.

Les adjuvants comprennent des polyols contenant au plus six atomes de carbone, et notamment polypropylène glycol, glycérol et sorbitol qui évitent un desséchement de la peau ou présentent un effet nourrissant. On utilisera également comme adjuvants des vitamines, notamment B6, C et PP, qui ont des effets bénéfiques sur la peau.

Les caractéristiques et avantages de l'invention ressortiront d'ailleurs de le description qui va suivre, à titre d'exemple, en référence aux dessins annexés dans lesquels:

la figure 1 représente un moulage électroconducteur sous forme de masque facial;

la figure 2A représente un moulage électroconducteur appliqué entre taille et cheville d'un sujet vu de face;

la figure 2B représente le moulage de la figure 2A mais vu de profil;

la figure 3 représente un détail de moulage avec un contact enrobé.

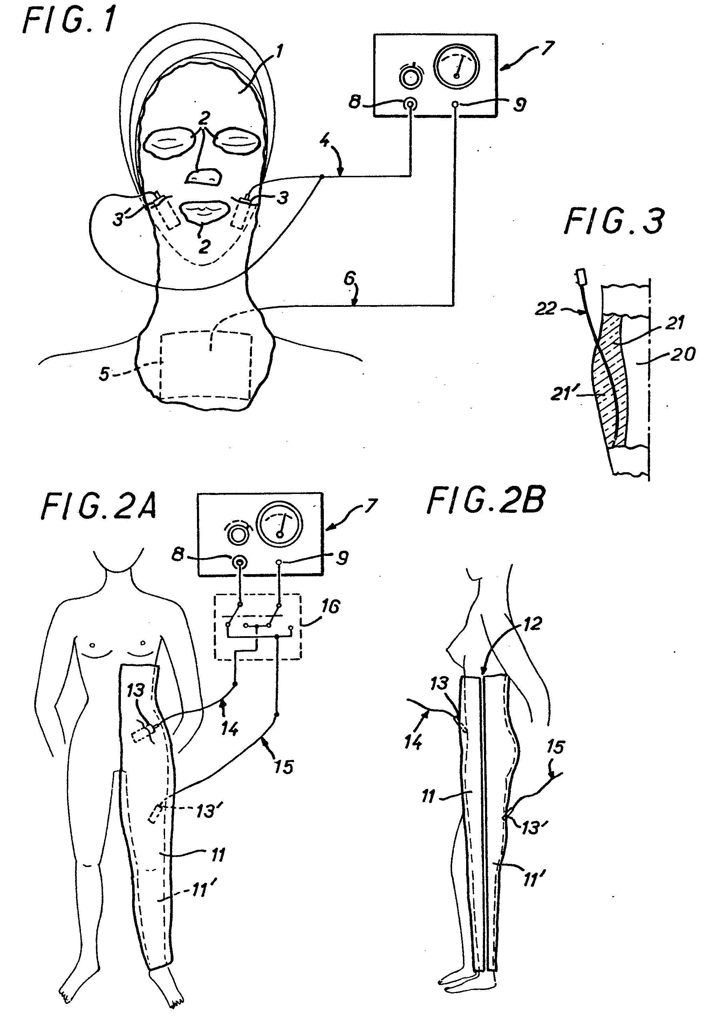

Selon la forme de réalisation choisie et représentée figure 1, un moulage électroconducteur 1, en plâtre dont l'eau de prise contient en solution du chlorure de calcium et des adjuvants, comme il sera décrit plus en détail aux exemples 1 et 2 ci-dessous, est appliqué sur le visage d'un sujet en descendant sur la partie antérieure du cou. Le moulage comporte des réserves 2 pour les yeux, les narines et les lèvres. Deux conducteurs métalliques 3 et 3' sont incorporés dans le moulage 1, au niveau des joues, et sont reliés à un conducteur commun 4. Une contre-électrode 5, constituée par un tampon de tissu absorbant imprégné d'un liquide conducteur, tel qu'un sérum physiologique glycériné, et doublé d'une feuille métallique souple vers l'extérieur, est appliqué sur le dos du sujet, entre les omoplates. La contre-électrode 5 est munie d'un conducteur de connexion 6. Un générateur 7 de courants de traitement thérapeutique ou esthétique, de type connu et prévu pour cet usage comporte une borne 8 de sortie de courants de traitement et une borne 9 de retour de courant. Sortie et retour des courants de traitement ne sont pas entendus ici par référence au sens conventionnel de circulation du courant d'une borne positive à une borne négative, mais par référence au sens des courants qui engendre une action thérapeutique ou esthétique en pénétrant dans la peau, de la borne de sortie 8 du générateur 7 en passant par le moulage électroconducteur 1, tandis que le courant sortant du corps par la contre-électrode 5 pour retourner au générateur 7 par la borne 9 ne produit pratiquement pas d'action physiologique.

On constitue le moulage en gâchant du plâtre dentaire avec de l'eau de prise contenant en solution du chlorure de calcium et des adjuvants, comme il sera expliqué plus en détail dans les exemples ci-dessous; lorsque le mélange gâché commence à se raffermir, on l'applique en couche de quelques millimètres d'épaisseur sur la peau du visage, préalablement ointe avec une composition ou crème connue, en ménageant des réserves 2 à l'emplacement des yeux, des narines et des lèvres, comme il est usuel dans l'application de masques traitants. L'onction avec la crème est destinée à adoucir le contact du moulage avec la peau, et également à concourir au traitement par apport de composants actifs.

On met en place les conducteurs 3 et 3' que l'on recouvre d'une surépaisseur de mélange gâché, en appuyant suffisamment pour assurer la compénétration des lite successifs de mélange gâché. On réalise alors le couplage des conducteurs 3 et 3' à la borne 8 par le conducteur 4, et l'on met en place la contre-électrode 5 reliée à la borne 9, des bandes adhésives assurant le bon contact de la contre-électrode 5 sur la peau. Lorsque la prise du mélange débute, ce qui est marqué par un échauffement du moulage, on met en service le générateur 7, réglé pour obtenir les conditions de traitement désirées. Comme on le précisera plus loin la composition du moulage est réglée pour ajuster le vitesse de prise, en fonction de la durée du traitement, de telle sorte que le traitement s'achève pour la prise complète. Après la fin du traitement, le moulage est détaché du visage par contraction de muscles telle que résultant d'un sourire. On peut constater que le moulage reproduit les détails de contour de la peau avec une grande finesse, ce qui atteste que le contact du moulage électroconducteur sur la peau au cours du traitement s'est étandu à toute la surface du masque.

Les électrodes cutanées destinées au traitement par courants électriques de parties étendues du corps peuvent être constituées comme représenté aux figures 2A et 2B, où le traitement intéresse la partie gauche du corps entre la taille et la cheville. Le moulage électroconducteur, dont la composition sera d'écrite plus en détail en référence à l'exemple 3, est constitué en deux parties, antérieure 11 et postérieure 11', séparées par une coupure 12, sensiblement dans un plan transversal au sujet. Sur la figure 2B, où le sujet est vu de profil, la coupure 12 est vue s'étendant de la taille à la cheville en suivant une ligne sur les faces externes de la hanche et de la jambe, mais on a compris que cette coupure 12 s'étend, invisible sur les figures, de la cheville au pubis en suivant les faces internes du mollet et de la cuisse. Dans la partie antérieure 11 du moulage électroconducteur est enrobé un conducteur 13, et dans la partie postérieure 11' est enrobé un conducteur 13', les conducteurs 13 et 13' étant prolongés respectivement par des câbles de connexion 14 et 15. Les câbles de connexion 14 et 15 sont reliés aux bornes 8 et 9 du générateur 7 à travers un inverseur 16, en sorte que les parties 11 et 11' du moulage puissent être couplées alternativement, soit respectivement aux bornes 8 et 9 de sortie et de retour de courant, soit aux bornes 9 et 8. Ainsi les parties 11 et 11' du moulage pourront jouer alternativement le rôle d'électrode cutanée et de contre-électrode, par manoeuvre de l'inverseur 16, les régions antérieure et postérieure sous-jacentes du corps étant traitées alternativement. Le gâchage du mélange de moulage, la préparation du moulage électroconducteur et la conduite du traitement sont analogues à ce qui a été décrit en référence à la figure 1.

Après la fin de la séance de traitement, les moulages sont enlevés. Il est fréquent que l'enlèvement du moulage occasionne sa destruction. De toute façon il est sans intérêt, notamment en raison du prix relativement modique des constituants du moulage, de vouloir le réutiliser pour une séance de traitement ultérieur, sans compter qu'il est peu probable qu'un moulage, réalisé antérieurement, soit susceptible d'épouser convenablement les contours de la peau par une remise en place ultérieure. Aussi les moulages sont considérés comme perdus, et sont systématiquement mis au rebut après une séance de traitement.

Comme on le voit mieux figure 3, l'enrobage des conducteurs de couplage 22 est réalisé en appliquant sur la peau 20 une première couche 21 de mélange de prise. Sur cette première couche 21 on applique le conducteur 22, en tresse métallique qui s'imprime dans la couche fraîche 21, et on recouvre l'extrémité de ce conducteur 22 par une seconde couche 21' de mélange de prise, en appuyant suffisamment pour que les couches 21 et 21' s'accrochent l'une sur l'autre en emprisonnant la tresse 22.

On va maintenant décrire des exemples de composition de moulage électroconducteur.

Exemple 1
Masque pour peau atone

On gâche 400 grammes de plâtre dentaire à durée de prise 5—10 minutes avec 200 grammes d'eau de prise à 25°C où l'on a fait dissoudre:

| | |
|---|---|
| Chlorure de calcium | 8 grammes |
| Glycérine | 5 g |
| Lipoprotéol* | 1 g |
| Vitamines B6 et C | 0,2 g |

*Le Lipoprotéol est un nom de marque désignant un tensio-actif contenant des acides aminés (protéines). La mousse qui se forme au gâchage par la présence du tensio-actif augmente la porosité et partant la souplesse du moulage au coura de la prise; les acides aminés, ionisables, vont migrer sous l'influence du courant de traitement vers la peau qui sera nourrie.

Cette composition de moulage présente une prise assez rapide accompagnée d'un échauffement marqué.

Avant l'application du moulage la peau du visage est ointe avec une creme nourrissante et anti-desséchante connue de composition suivante:

| | |
|---|---|
| Cire d'abeille | 24 grammes |
| Alcool cétylique | 56 g |
| Amphisol (nom de marque) | 8 g |
| Vaseline épaisse | 80 g |
| Lanoline | 40 g |
| Alcool oléique | 40 g |
| Myristate d'isopropyle | 80 g |
| Nipastat (nom de marque) | 2,4 g |
| Butyl hydroxy toluène | 0,4 g |
| Eau | 410 g |
| Borax | 8 g |
| Extrait de tilleul | 10 g |
| Phénonip (nom de marque) | 2.4 g |
| | pour 760 grammes environ. |

Exemple 2
Masque pour peaux sensibles

On gâche 400 grammes de plâtre dentaire à durée de prise 10—15 minutes avec 200 grammes d'eau de prise à 18°C, dans laquelle on a dissous préalablement:

| | |
|---|---|
| Chlorure de calcium | 16 grammes |
| Propylène-glycol | 10 g |
| Lipoprotéol (nom de marque) | 1 g |
| Vitamines PP et C | 0,2 g |

Le mélange gâché présente une durée de prise sensiblement allongée, et une élévation de température lors de la prise sensiblement moindre que le melange de l'exemple 1, en raison notamment de la teneur relativement élevée en chlorure de calcium. Le Lipoprotéol a le même effet que dans l'exemple 1, et le propylène-glycol agit comme le glycérol de l'exemple précédent. Les vitamines sont choisies adaptées au type de peau considéré.

Avant l'application du masque, la peau du visage sera ointe avec une crème convenable, de composition connue comme suit:

| | |
|---|---|
| Stéarate de butyle | 80 grammes |
| Huile de vaseline épaisse | 49,6 g |
| Dodécanol | 80 g |
| Alcool cétylique | 32 g |
| Amphisol (nom de marque) | 24 g |
| Huile d'amandes douces | 32 g |
| Butyl hydroxy toluène | 0,8 g |
| Nipastat (nom de marque) | 2,4 g |
| Eau | 350 g |
| Sorbitol | 48 g |
| Glycérine | 80 g |
| Méthylrutine | 0,8 g |
| Composé floral hamamélis | 24 g |
| Azulène | 0,8 g |
| Phénonip (nom de marque) | 2 g |
| Parfum | 1,6 g |
| | pour environ 800 grammes. |

Exemple 3
Moulage électroconducteur pour le corps

On gâche 4000 grammes de plâtre dentaire à durée de prise 10—15 minutes avec 2000 grammes d'eau à 30°C contenant en solution:

| | |
|---|---|
| Clorure de calcium | 100 grammes |
| Sorbitol | 50 g |
| Lipoprotéol | 5 g |
| Sulfate de galactanne | 5 g |

Le sorbitol, qui est un itol dérivé d'un hexose, est donc un polyol à six atomes de carbone, et posséde un pouvoir anti-desséchant analogue à celui des glycol et glycérol des exemples précédents, joint à un pouvoir nourrissant dû à sa formule voisine de celle d'un sucre. Le Lipoprotéol est moussant et apporte ses protéines. Le sulfate de galactanne joue en rôle amincissant.

Le moulage effectué avec ce mélange reste assez souple pour supporter pendant au moins 15 minutes les mouvements excito moteurs (réactions musculaires aux courants électriques de traitement) sans cassure.

Avant application du moulage le corps sera oint dans les régions intéressées avec un produit anti-desséchant et traitant de composition connue, telle que:

| | |
|---|---|
| Eau | 1470 grammes |
| Glycérine | 20,8 g |
| Bronidox (nom de marque) | 0,24 g |
| Phénonip (nom de marque) | 1 g |
| Sulfate de galactanne | 48 g |
| Carbopol (nom de marque) | 40 g |
| Colorant | 0,8 g |
| Parfum | 1,34 g |
| Triéthanolamine | 43,8 g |
| | pour environ 1600 grammes. |

Des essais complémentaires ont permis de vérifier que suivant la teneur an chlorure de calcium de l'eau de prise, variant entre 3 et 10% en masse par rapport à l'eau, la vitesse de prise variait, diminuant lorsque la teneur en chlorure de calcium était augmentée; parallèlement

**0 004 514**

l'échauffement du moulage au cours de la prise varie comme la vitesse de prise. La durée de traitement doit être du même ordre du grandeur que la durée de prise complète pour que la conductivité du moulage, sa souplesse relative, et la liberté de migration des adjuvants dissous dans l'eau de prise restent suffisantes pour assurer l'efficacité du traitement, en sorte qu'il est clair que la durée prévue du traitement conditionne la vitesse de prise et donc la concentration en chlorure de calcium de l'eau de prise, qui sera précisée par des essais de routine. Par ailleurs les méthodes de traitement échappent au cadre de la présente invention et relèvent de l'art médical.

**Revendications**

1. Electrode cutanée pour application sur la peau du corps humain de courants électriques de traitement thérapeutique ou esthétique, en association avec une contre-électrode, électrode et contre-électrode étant couplées respectivement à deux bornes d'un générateur de courants électriques de traitement, électrode du genre que l'on prépare sous forme d'une composition à appliquer directement sur la peau, se solidifiant après mise en place et constituée par un mélange d'un liant plastique et d'un additif conducteur d'électricité, caractérisée en ce que le liant plastique est constitué d'une poudre susceptible de prise avec l'eau, et d'eau en quantité supérieure à celle qui se combine avec la poudre durant la prise, l'additif est un sel dissous dans l'eau ajoutée pour la prise, la composition en se solidifiant formant un moulage électroconducteur qui épouse et maintient la forme du corps.

2. Electrode selon la revendication 1, caractérisée en ce qu'au moins un contact métallique de couplage (3, 3') au générateur (7) est incorporé par enrobage dans le moulage.

3. Electrode selon la revendication 1 ou la revendication 2, caractérisée en ce que, ladite poudre étant un plâtre de catégorie dentaire, l'eau de prise, utilisée en quantité voisine en masse de la moitié de celle du plâtre, contient en solution entre 3 et 10% en masse de chlorure de calcium.

4. Electrode selon une quelconque des revendications 1 à 3, caractérisée en ce que l'eau de prise contient en outre un produit tensio-actif protéiné et des adjuvants actifs sur la peau.

5. Electrode selon la revendication 4, caractérisée en ce que les adjuvants comprennent un polyol contenant au plus six atomes de carbone.

6. Electrode selon la revendication 5, caractérisée en ce que ledit polyol est choisi dans le groupe comprenant polypropylene-glycol, glycérol et sorbitol.

7. Electrode selon une quelconque des revendications 3 à 6, caractérisée en ce que les adjuvants comprennent au moins une vitamine choisie dans le groupe comprenant les vitamines B6, C et PP.

8. Electrode selon une quelconque des revendications 1 à 7, caractérisée en ce que ledit moulage est divisé en deux parties (11, 11') par une coupure d'isolement (12), chaque partie étant couplée respectivement à une des deux bornes (8, 9) du générateur (7) de courants.

9. Electrode selon la revendication 8, caractérisée en ce que les deux parties de moulage (11, 11') sont couplées aux bornes respectives (8, 9) du générateur à travers un moyen d'inversion (16).

**Patentansprüche**

1. Hautelektrode, die elektrische Ströme zur therapeutischen und ästhetischen Behandlung auf die Haut des menschlichen Körpers leitet in Verbindung mit einer Gegenelektrode, wobei die Elektrode bzw. die Gegenelektrode über zwei Anschlußklemmen mit einem elektrischen Stromgenerator zur Behandlung angeschlossen sind und wobei die Elektrode unter Formung einer Mischung vorbereitet wird zum unmittelbaren Auftragen auf die Haut, auf der die Mischung nach dem Anbringen erstarrt und wobei die Mischung sich zusammensetzt aus einem Kunststoffbindemittel und einem elektrisch leitenden Additif, dadurch gekennzeichnet, daß das Kunststoffbindemittel aus einem mit Wasser abbindefähigen Pulver besteht, daß die Wassermenge größer ist als die Menge, die sich mit dem Pulver während des Abbindens verbindet, daß das Additif ein in dem Wasser gelöstes, zum Abbinden hinzugefügtes Salz ist und daß die Zusammensetzung während des Erstarrens eine elektrisch leitende Form bildet, die sich der Körperform anpaßt und die Form beibehält.

2. Elektrode nach Anspruch 1, dadurch gekennzeichnet, daß wenigstens ein metallischer Kontakt (3, 3') zur Verbindung mit dem Generator (7) in Form einer Umhüllung in der Form eingebaut ist.

3. Elektrode nach Anspruch 1 oder 2, dadurch gekennzeichnet, daß das Pulver ein Gips ist, wie er in der Dentalpraxis verwendet wird, und daß das Abbindewasser, das in einer Menge von etwa der Hälfte der Masse des Gipses verwendet wird, in Lösung zwischen 3 und 10 Massen-% Kalziumchlorid enthält.

4. Elektrode nach einem der Ansprüche 1 bis 3, dadurch gekennzeichnet, daß das Abbindewasser außerdem ein Produkt eines oberflächenaktiven Proteins und hautaktive Zusatzstoffe enthält.

5. Elektrode nach Anspruch 4, dadurch gekennzeichnet, daß die Zusatzstoffe ein Polyalkohol mit höchstens 6 Kohlenstoffatomen enthält.

6. Elektrode nach Anspruch 5, dadurch gekennzeichnet, daß der Polyalkohol ausgewählt ist aus der Gruppe, welche Polypropylenglykol, Glyzerin und Sorbit enthält.

7. Elektrode nach einem der Ansprüche 3 bis 6, dadurch gekennzeichnet, daß die Zusatzstoffe wenigstens ein Vitamin aus der Gruppe der Stoffe mit den Vitaminen B6, C und PP enthalten.

8. Elektrode nach einem der Ansprüche 1 bis 7, dadurch gekennzeichnet, daß die Form auf zwei Teile (11, 11') aufgeteilt ist durch einem isolierenden Einschnitt (12), und daß jedes Teil jeweils über eine Anschlußklemme (8, 9) mit dem Stromgenerator (7) verbunden ist.

9. Elektrode nach Anspruch 8, dadurch gekennzeichnet, daß die beiden Formteile (11, 11') mit den Anschlußklemmen (8) bzw. (9) des Generators über eine Umwandlervorrichtung (16) verbunden sind.

## Claims

1. Cutaneous electrode for application of electric current for therapeutic or aesthetic treatment to the skin of a human being, in association with a counterelectrode, the electrode and counterelectrode being respectively coupled to two terminals of a treatment electric current generator, the electrode being of the kind that is prepared in the form of a composition to be applied directly to the skin, solidifying after positioning and comprised of a mixture of a plastic binder and an electrically conductive additive, characterised in that the plastic binder is comprised of a powder settable with water, and of water in an amount greater than that which will combined with the powder during setting, the additive is a salt dissolved in water added for setting, the composition forming, by solidifying, an electroconductive cast which mates with and keeps the shape of the body.

2. Electrode according to claim 1, characterised in that at least one metal contact (3, 3') for connecting to the generator (7) is incorporated by embedding in the cast.

3. Electrode according to claim 1 or claim 2, characterised in that, the said powder being a dental grade plaster, the water for setting present in an amount equal to about half the weight of the plaster, contains in solution between 3 and 10% by weight of calcium chloride.

4. Electrode according to any one of claims 1 to 3, characterised in that the water for setting further contains a proteinaceous surface-active agent and adjuvants active on the skin.

5. Electrode according to claim 4, characterised in that the adjuvants comprise a polyalcohol containing at most six carbon atoms.

6. Electrode according to claim 5, characterised in that the said polyalcohol is selected from a group comprising polypropylene-glycol, glycerol and sorbitol.

7. Electrode according to any one of claims 3 to 6, characterised in that the adjuvants comprise at least one vitamin selected from the group comprising vitamins B6, C and PP.

8. Electrode according to any one of claims 1 to 7, characterised in that the said cast is divided into two parts (11, 11') by an isolating break (12), each part being coupled respectively to one of the terminals (8, 9) of the current generator (7).

9. Electrode according to claim 9, characterised in that the two cast parts (11, 11') are coupled to the respective terminals (8, 9) of the generator through inversion means (16).

0 004 514

**FIG.1**

**FIG.3**

**FIG.2A**

**FIG.2B**

1